# EUROPEAN PATENT APPLICATION

(11) **EP 2 335 636 A2**
(43) Date of publication of application: **22.06.2011**
(21) Application number: 10195010.3
(22) Date of filing: 14.12.2010
(51) Int. Cl.: A61B 19/02, A61L 2/20, B65B 55/02, A61B 17/00, A61B 17/34, A61B 19/00

(54) **Sterilizable package having breathable membrane for the packaging of medical devices**

(30) Priority: 15.12.2009 US 637995
(71) Applicant: Amcor Flexibles, Inc., Mundelein, IL 60060 (US)
(72) Inventor: Dworak, Adam, NORTHBROOK, IL 60062 (US)
(74) Representative: Corret, Hélène

(57) **Abstract**

The invention provides a sterilizable package having a support member and a sheet material that either forms a lidding for the support member or a pouch in which the support member is disposed. The sheet material comprises a barrier material and includes breathable membrane comprising a breathable material that is pervious to moisture and gases, and impervious to microorganisms. The breathable membrane allows a sterilizing gas to be introduced into the sterilizable package. The breathable membrane is disposed towards a central portion of the sheet material and is spaced apart from any seams joining the sheet material to the support member or seams defining the pouch. In one embodiment, a sterilized article is disposed in the interior of the sterilizable package.

## Description

### FIELD OF THE INVENTION

The present invention is directed to a package for sterilizing articles, and more particularly, a package for the packaging and sterilization of medical devices.

### BACKGROUND OF THE INVENTION

Generally, it is desirable to sterilize medical instruments and devices after packaging and prior to being sent to the end user. Common forms of sterilization include irradiation; autoclaving, and treatment with a sterilizing gas, such as ethylene oxide. Typically, treatment with a sterilizing gas is used to sterilize many such instruments and devices. For example, one conventional form of package for sterilizing medical devices, such as a stent, comprises a polymeric sheet and a sheet of a breathable material, such as Tyvek®, that are attached to each other along their peripheral edges to form seams defining a pouch. The breathable material is typically a sheet material that is a microbe barrier and is gas pervious. Another common package for packaging of medical devices is a blister-type package in which a sheet of breathable material it sealed to the peripheral edge of a tray to form a lidding. After an article has been sealed in one of these types of packages, a sterilizing gas can then be introduced into the interior of the package through the breathable membrane. These types of packaging have several disadvantages.

In particular, breathable materials, such as Tyvek®, can typically only be heat sealed below a given temperature due to melting of the material, which limits the speed at which such packages can be manufactured. Opening of the package by the end user can also present some issues. Tyvek®, which is widely used as a microbe barrier material, is a nonwoven sheet material made of individual fibers that are thermally bonded to each other to form a coherent fabric. Opening of a package that includes a breathable material along a seam may result in the creation of small fibers that may be deposited on the sterilized article. In addition, seams comprising a breathable material and a polymeric material are typically weaker than a seam that is between two polymeric materials.

Such conventional packaging for stents and other medical devices/instruments are generally considered inefficient and wasteful of material and labor. Accordingly, there is a need for a more cost and labor effective method for the packaging and sterilization of medical devices and instruments.

### BRIEF SUMMARY OF THE INVENTION

In one embodiment, the present invention is directed to a sterilizable package having a support member that is disposed in a pouch comprising a barrier sheet material that includes a breathable membrane. The breathable membrane comprises a breathable material that is pervious to gases, and substantially impervious to microorganisms. The breathable membrane allows a sterilizing gas to be introduced into an interior space of the sterilizable package. In one embodiment, the sterilizable package includes a pouch in which the sheet material is folded and sealed to itself along opposing side seams and a longitudinal seam to define the pouch. The breathable membrane is preferably disposed towards a central portion of the sheet material and is not present where the sheet material is sealed to itself or another sheet material. It has been discovered that by positioning the breathable material towards a central portion of the sheet material and away from the seams forming the pouch, the strength of the seams is increased in comparison to packaging in which a breathable material is part of the seam. Further, by not including a breathable material as part of the seam, fiber tearing during opening of the package can be avoided. In addition, by spacing the breathable membrane away from the seam forming the package, the present invention makes it possible to use less breathable material, in the lidding or the pouch while still being able to maintain a desired level of sterilization.

In a preferred embodiment, the sheet material includes an opening formed therein for providing communication between the outside and interior of the sterilizable package. A breathable membrane comprising a microbe barrier, gas permeable sheet material is disposed on an inner or outer surface of the sheet material and covers the opening. The breathable membrane includes a peripheral edge overlying the sheet material and that is spaced from the seams forming the pouch. A continuous seam is located at or adjacent to the peripheral edge of the breathable membrane and joins the membrane to the sheet material.

In one embodiment, an article to be sterilizable is introduced into and sealed within the sterilizable package. Thereafter, a sterilizing gas can be introduced into the sealed package containing the article via the breathable membrane.

In a further aspect of the invention, a sterilizable package is provided comprising a support member to which the sheet material having the breathable membrane is affixed to form a lidding of the sterilizable package. As in the embodiment discussed above, the breathable membrane is not part of the seal between the support member and the sheet material.

### BRIEF DESCRIPTION OF THE SEVERAL VIEWS OF THE DRAWING(S)

Having thus described the invention in general terms, reference will now be made to the accompanying drawings, which are not necessarily drawn to scale, and wherein:
FIG. 1 is a perspective view of a sterilizable package in accordance with one embodiment of the invention;
FIG. 2 is a cross-sectional side view of the pouch of FIG. 1 taken along line 2-2 of FIG. 1;
FIG. 3 is a cross-sectional side view of the pouch of FIG. 1 taken along line 3-3 of FIG. 1;
FIG. 4 is a perspective view of a sterilizable package in accordance with one embodiment of the invention in which the sheet material having the breathable membrane forms a lidding component in the sterilizable package;
FIGS. 5A and 5B illustrate examples the sheet material in which it is provided in a roll, and in which the sheet material includes a plurality of spaced apart breathable membranes that can be used to form sterilizable packages in accordance with the present invention; and
FIG.6 illustrates an exemplary HFFS packaging application that can be used to prepare sterilizable packages in accordance with the claimed invention.

### DETAILED DESCRIPTION OF THE INVENTION

The present invention now will be described more fully hereinafter with reference to the accompanying drawings, in which some, but not all embodiments of the inventions are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

The present invention provides sterilizable packages that include a sheet material having a breathable membrane through which a sterilizing gas can be introduced into the interiors of the packages for sterilizing the contents of the package. The breathable membrane comprises a breathable material through which a gas, such as a sterilizing gas can be introduced into the interior of the package. The present invention also provides for sterilizable packages that are formed from the sheet material, and for processes of making such sterilizable packages. In particular, the sheet material having the breathable membrane can be used in a variety of packaging applications including, but not limited to horizontal-form-fill-seal (HFFS), vertical-form-fill-seal (VFFS), vacuum skin packaging (VSP), flow wrap packaging, thermoforming packaging, and the like. The barrier sheet material having the breathable membrane may also be used as a lidding for packages comprising a support member (e.g., tray) to which the sheet material has been adhered. For example, in one embodiment, the sheet material may be heat sealed to a support member, such as a tray, to form a sealed sterilizable package.

In addition, sterilazable packages in accordance with the present invention can also be used in packaging applications where a change in pressure may result in rupture of the package. For example, in the case of air transport of a sealed package, a change in pressure may cause the seams of the package to rupture upon a change in pressure if the atmosphere within the package cannot escape. In the present invention, the breathable membrane provides a means from which air may ingress or egress out of the interior of the package. As a result, the sterilazable packages of the present invention may advantageously be used in packaging applications in which the sealed package is transported/shipped via an air carrier.

Sterilizable packages in accordance with the present invention can be use to package a variety of different articles including medical instruments and devices. For example, the sterilizable package can be used to package and sterilize scalpels, scissors, sutures, forceps, retractors, blades, clamps, stents, both treated and untreated, catheters, etc.

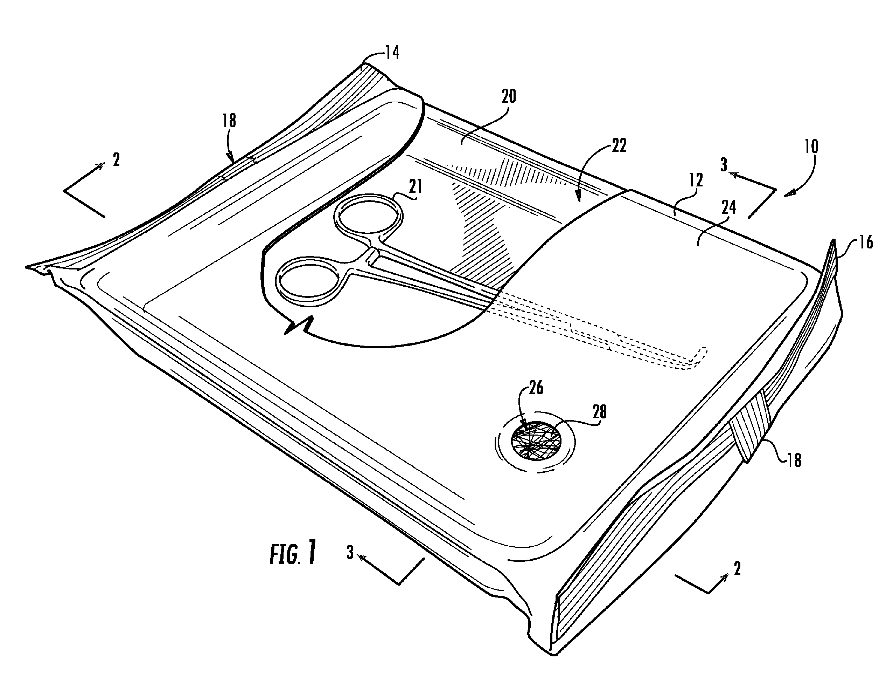

With reference to FIG. 1, a sterilizable package in accordance with the present invention is illustrated and broadly designated by reference number **10.** As shown, the sterilizable package **10** includes an outer pouch **12** having two opposing side seams **14, 16,** and a longitudinal seam **18** that extends longitudinally between side seams **14, 16.** A support member **20** for supporting an article **21** thereon is disposed with in the interior space **22** of the pouch.

The pouch is formed from a polymeric sheet material **24** that is substantially impervious to microorganisms. The pouch includes one or more breathable membranes **26** comprising a breathable material **28** through which a gas, such as a sterilizing gas, can be transmitted into and out of the pouch. The breathable membrane preferably comprises a sheet material that permits the passage of gases while limiting the passage of undesirable materials, such as microorganisms. Suitable materials for the breathable membrane include nonwovens, medical grade paper, microbial barrier membranes, and other porous materials that limit the passage of microbes. In the context of the invention, the term "pouch" is used in a generic sense and should be recognized to include, sacks, bags, satchels and the like.

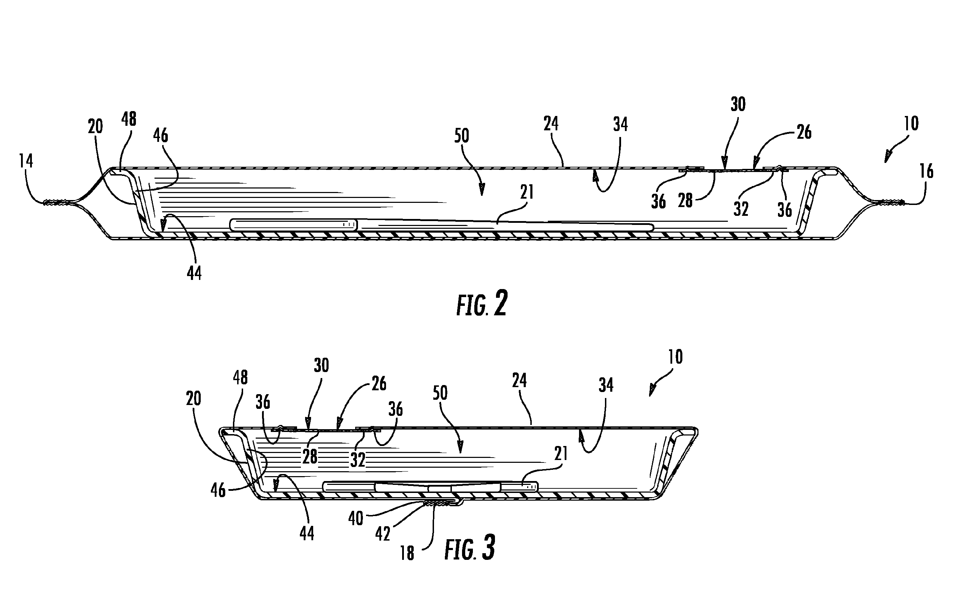

Turning to FIG. 2, a cross-sectional view of the sterilizable package **10** taken along line 2-2 of FIG. 1 is illustrated. The breathable membrane is formed from an opening **30** in the sheet material which provides communication between the interior of the package and the surrounding environment. The breathable membrane **26** comprises microbe barrier, gas permeable, sheet material **28** that is disposed on the sheet material **24** and covers the opening. The breathable membrane has a peripheral edge **32** that overlies the sheet material **24** and is spaced from the opposing side seams **14, 16** and longitudinal seam **18** of the pouch. In the illustrated embodiment, the opening 30 and the breathable membrane **26** are positioned towards a central portion of the sheet material **24.** That is, the breathable membrane **26** is positioned away from side seams **14, 16** and longitudinal seam **18.** The breathable material **28** is joined to an inner or outer surface **34** of the sheet material **24** along a continuous seam **36** that is located towards or adjacent to the peripheral edge **32** of the breathable membrane **26.** Preferably, the breathable membrane is joined to the sheet material with a heat seal. The heat seal can be in the form of a peel seal or a destruct seal. In one embodiment, the opening **30** in the sheet material **24** and the breathable membrane **26** are circular, and are joined to each other by a continuous seam **36** that is located at or adjacent the peripheral edge **32** of the breathable membrane.

The opening **30** can be made in the sheet material in a variety ways as known in the art, including punching, die cutting, cutting, and the like.

The breathable membrane comprises a material that is a barrier to microorganisms, but is permeable to gases including oxygen, carbon dioxide, and various sterilization gases. Suitable materials for the breathable material may include paper and nonwoven sheet materials. Suitable nonwoven sheet materials include spunbond nonwoven fabrics such as Typar® and Reemay® fabrics from Fiberweb Inc., and nonwoven fabrics formed of flash-spun polyethylene strands, such as a nonwoven sheet material sold by E.I. Du Pont de Nemours and Company under the trademark Tyvek®.

The breathable membrane is positioned on the sheet material, and hence the pouch, so that it is spaced away from the seams forming the pouch (e.g., side seams **14, 16** and longitudinal seam **18).** By positioning the breathable membrane towards a central portion of the sheet and away from the seam(s) forming the pouch, the strength of the seams can be greatly improved. In addition, since the breathable membrane is not part of the seal between an opposing sheet material or tray, tearing of the breathable membrane, which may result in fibers being deposited on the packaged article, during opening of the package can be limited or avoided.

The breathable membrane has a surface area that is generally from about 10 to 60 % larger than the surface area of the opening formed in the sheet material, and in particular, from about 20 to 50 % larger than the surface area of the opening formed in the sheet material. For example, in the illustrated embodiment, the opening has a diameter that is about 3 inches, whereas the breathable membrane has a diameter that is about 5 inches. In this embodiment, the large sized breathable membrane helps to provide more freedom in the manufacturing process so that the breathable membrane does not have to be precisely positioned over the opening prior to sealing the breathable membrane to the sheet material.

The breathable membrane generally overlies between about 2 and 25 percent of the surface area of the sheet material. In one embodiment, the breathable membrane overlies between about 5 and 10 percent of the surface area of the sheet material, and more typically between about 5 and 15 percent of the surface area of the sheet material. It should be understood that the size and location of the breathable membrane is not limited to any particular configuration and that the position and size can be selected to meet the particular requirements of the end user. Additionally, the position and size of the breathable membrane can be selected to optimize the sterilization process.

In the figures, the sheet material is depicted as having a single breathable membrane having a generally circular shape. However, it should be recognized that the present invention is not limited to any particular number, shape or size of the breathable membrane and that the sheet material can include multiple breathable membranes of varying shapes and sizes. It should also be recognized that the opening in the sheet material is not limited to any particular size or configuration. For example, the opening may comprise one or more slits or smaller openings depending on the design and/or end application.

In embodiments in which the breathable membrane has a circular shape, the diameter of the breathable membrane can generally range from about 1 to 10 inches, with a diameter of about 2 to 5 inches being preferred.

As can best be seen in FIG. 3, the pouch **12** has a generally tube-like structure in which the pouch is formed from a single sheet material **24.** In this embodiment, sheet material **24** includes longitudinal side edges **40, 42** extending along the length of the sheet material that are sealed to each other along longitudinal seam **18.** The tube-like structure of pouch is created by folding the sheet material along its length so that the two opposing longitudinal edges may be adhered to each other to form a longitudinal seal along the length of the bag. For example, sheet material **24** is folded in such a manner that the inner surface **34** of the sheet material adjacent to the longitudinal side edges **40, 42** is superimposed and heat sealed to itself to form longitudinal seam **18.** This type of seam is commonly referred to as a fin seal. It should be recognized that other seals may be used in the practice of the invention including lap seals, reverse fin seals, and the like. Longitudinal seam **18** extends longitudinally along the length of the pouch between opposite side seams **14, 16**.

Alternatively, the pouch **12** can be prepared from two separate sheets of material that are oriented in a face-to-face relation, and are sealed to each other along opposing edges to define a pouch having an interior space for receiving the support member therein.

Preferably, the inner surface **34** of the sheet material **24** comprises a heat sealable material. In the particular embodiment illustrated, the sheet material is made from a heat sealable material and the opposing ends of the pouch are sealed by producing a fusion bond or seal between contacting interior surfaces of the sheet material **24** using pressure and heat or ultrasonic energy as is well known. Although referred to herein as "heat seals", it should be understood that this term is intended to apply both to seals formed by heating the contacting surfaces with a heated anvil or platen, as well as to heating and fusion produced by other methods, such as application of ultrasonic energy. As discussed in greater detail below, suitable polymeric sheet materials for use in the present invention may include polyolefins, such as polyethylene and polypropylene, polyesters, nylons, etc.

As noted above, the support member **20** provides a component of the sterilizable package on or in which the article to be sterilized is disposed. The support member of the sterilizable package may be flat or substantially planar but is preferably formed in the shape of a tray. Preferably, the support member includes a downwardly formed cavity and an upper flange, wherein the product support surface is defined by the downwardly formed cavity and wherein the upper flange is the periphery of the support member. In this regard, FIG. 2 illustrates the support member having a support surface **44** on which the packaged article is supported and a plurality of interconnected sidewalls **46** defining cavity **50.** Preferably, each of the sidewalls culminates in flange **48** that extends peripherally around the support member.

The support member may be semi-rigid but is preferably rigid, and is not limited to any particular shape or configuration. For example, the support member can be rectangular, round, oval, etc. Similarly, flange **48** may have any desired shape or design, including a simple, substantially flat design which presents a single sealing surface as shown, or a more elaborate design which presents two or more sealing surfaces. In the case of HFFS and VFFS packaging, it may be desirable for the support member to have a rectangular or square shape.

Suitable materials from which support member **20** can be formed include, without limitation, polyvinyl chloride, polyethylene terephthalate, polystyrene, polyolefins such as high density polyethylene or polypropylene, paper pulp, nylon, polyurethane, etc. The support member may be foamed or non-foamed as desired, and preferably provides a barrier to the passage of oxygen therethrough. In some embodiments, the support member **20** may be formed from a material which itself provides a barrier to the passage of oxygen, e.g., vinylidene chloride copolymer, nylon, polyethylene terephthalate, ethylene/vinyl alcohol copolymer, etc. Alternatively, support member **20** may have a substantially gas-impermeable sealant film laminated or otherwise bonded to the inner or outer surface thereof.

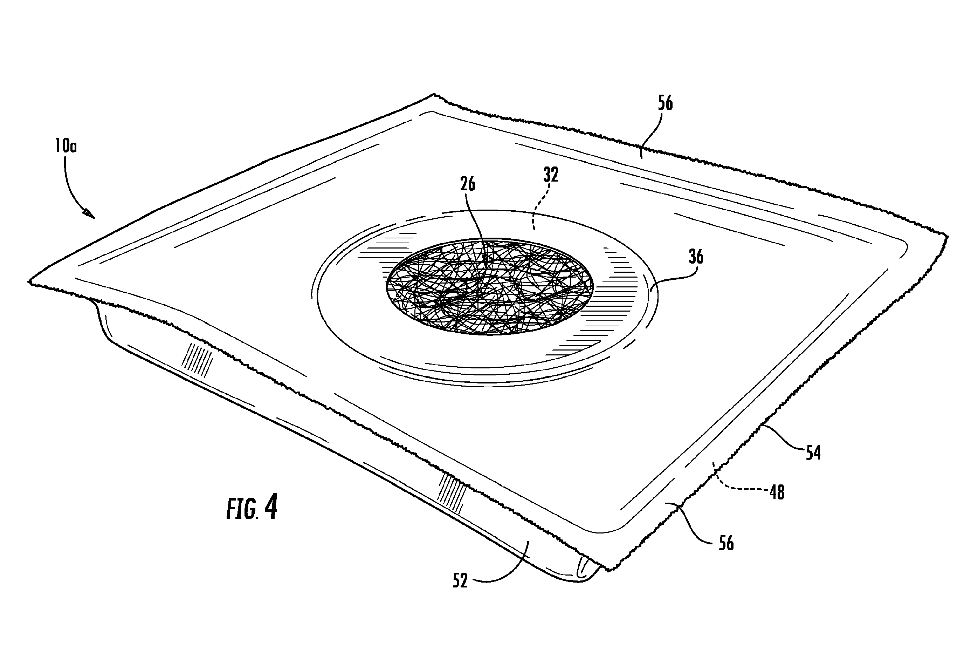

In one embodiment, the support member comprises a thermoplastic material that is thermoformed into a desired shape. In this regard, FIG. 4 illustrates a variation of a sterilizable package **10a** comprising a support member **52** (e.g., tray) to which the sheet material **24** has been adhered to form a lidding **54.** In this embodiment, sheet material **24** is adhered to flange **48** to sealably close the package. Preferably, sheet material is affixed to the support member **52** with a heat seal. The sterilizable package includes heat seal **56** joining the sheet material and support member to each other. Heat seal **56** extends around the periphery of the sheet material where the sheet material is affixed to the support member.

In embodiments in which the support member is thermoformed, the support member may be thermoformed in-line with the packaging operation or provided preformed. Depending on the product being packaged and the ultimate end-use application the support member may be gas permeable or substantially gas impermeable. Additionally, depending on the composition of the inner surface of the sheet material (i.e., the surface affixed to the support member) the support member may comprise a heat sealable material. For example, the support member may include a sealant film for heat sealing the support member to sheet material **24.**

As in the sterilizable package discussed above, the breathable membrane **26** is positioned on the sheet material so that it is spaced away from seal **56** so that the breathable membrane is not part of seal **56.** In a preferred embodiment, the support member **52** comprises a thermoplastic material has been thermoformed into a tray as is known in the art.

Seal **56** can be made peelable so that the lidding **54** can be easily removed during use. In peelable applications, the peal strength of seal **56** is typically about 0.5 to **4** pounds per inch. In contrast to prior art packaging applications in which the lidding or a sheet of the pouch comprises a sheet of the breathable material, such as Tyvek®, the present invention makes it possible to use less breathable material, in the lidding or the pouch while still being able to maintain the same sterilizable properties, and also makes it possible to prepare peelable applications that do not need a coating on the breathable material, such as a coated Tyvek®.

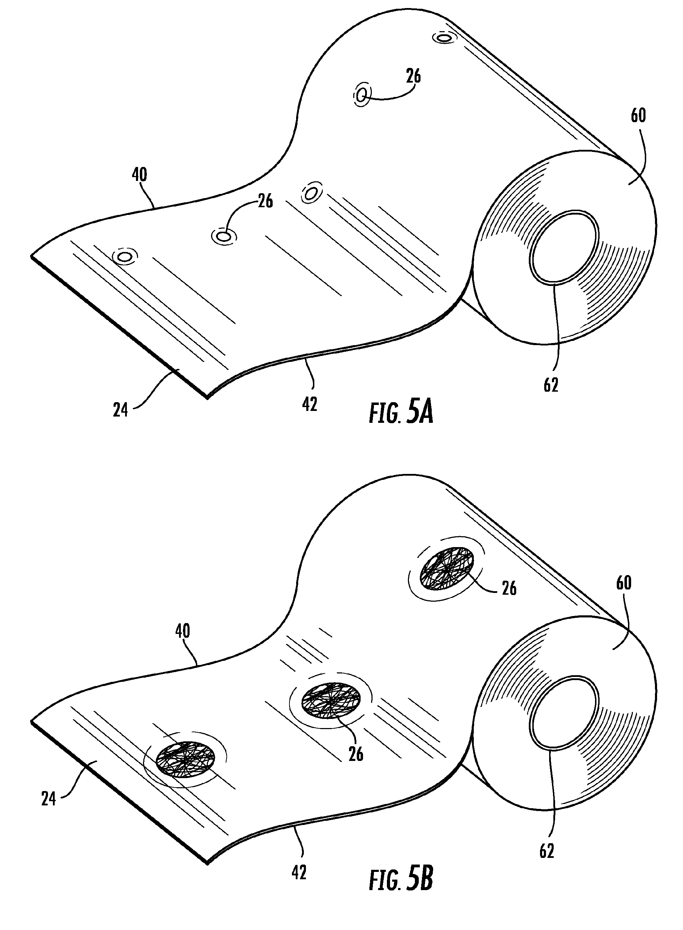

FIGS. 5A and 5B illustrate rolls **60** of sheet material **24** that can be used in preparing sterilizable packages in accordance with the invention. As shown, sheet material **24** is wound around a central core **62** to form a roll of sheet material. The size, shape, and configuration of the roll **60** and core **62** can be adjusted so that the roll of sheet material **24** can be used interchangeably with commercially available packaging equipment. Sheet material can include a plurality of spaced apart breathable membranes **26.** The spacing of the breathable membranes can be selected so that the packages prepared therefrom can have a desired number of breathable membranes in a desired location of the packaging. The spacing of the breathable membranes can also be selected so that the roll stock **60** can be used in automated packaging processes. As can be seen in FIGS. 5A and 5B, the size of the breathable membranes can also be varied. For example, in FIG. 5A the breathable membranes have a relatively small diameter in comparison to the breathable membranes depicted in FIG. 5B.

As discussed above, the one or more breathable membranes are positioned at locations on the sheet materials so that upon forming a package from the sheet material, the breathable material of the breathable membrane does form part of the seal forming the packaging. Preferably, the breathable membranes **26** are positioned towards the center of the sheet material and away from the longitudinal side edges **40, 42** of the sheet material. For example, the breathable membranes **26** are generally spaced away from the side edges **40, 42** by at least 1 cm, with a spacing of at least 2 cm being preferred, and a spacing of at least 3 cm being even more preferred.

Sheet materials in accordance with the present invention may be used as stock roll for standard equipment adapted to fabricate bags, pouches, or other dilatable products, by slitting, sealing, folding and whatever other operations are dictated by the form of the product. In particular, the sheet material is particularly useful in vertical form fill and seal (VFFS), horizontal form fill and seal (HFFS) packaging processes, lidding in thermoforming applications, vacuum seal packaging, and the like.

The sheet material itself, comprises a flexible polymeric film having microbe barrier properties. In a preferred embodiment, the sheet material is a polymeric film having liquid, moisture vapor, and gas barrier properties. In one embodiment, polymeric films for as the sheet material have an oxygen vapor transmission rate that is less than about 1 cc/m²/day, and in particular less than about 0.5 cc/m²/day, and more particularly less than about 0.2 cc/m²/day as measured according to ASTM test method 3985.

Sheet material **24** may be in the form of a mono-layer, multi-layer film, or laminate. In one embodiment, the sheet material **24** comprises a multilayer film including one or more polymeric or other layers composed of compositions selected to impart specific properties to the film. In one embodiment, the sheet material has barrier properties. Suitable components the sheet material may include metallic foil, such as aluminum foil, and metallized films, such aluminized films, aluminum oxide films (AlOx), silicon oxide films (SiOx), and films comprising polychlorotrifluoroethylene (PCTFE) such as Aclar®. The sheet materials may also include polymeric components having barrier properties, such as ethylene/vinyl alcohol copolymer ("EVOH"), polyvinyl alcohol ("PVOH"), vinylidene chloride polymers ("PVdC"), polyalkylene carbonate, polyester (e.g., PET, PEN), polyacrylonitrile ("PAN"), and polyamides.

Useful polyamides may include polyamide 6, polyamide 9, polyamide 10, polyamide 11, polyamide 12, polyamide 66, polyamide 610, polyamide 612, polyamide 61, polyamide 6T, polyamide 69, copolymers made from any of the monomers used to make two or more of the foregoing homopolymers (e.g., copolyamide 6/12, polyamide 12, copolyamide 66/69/6I, copolyamide 66/610, copolyamide 6/66, and copolyamide 6/69), and blends of any of the foregoing homo- and/or copolymers.

Polymeric films suitable for use as sheet material may include one or more additional layers that impart desired properties to the film. For example, the sheet material may include one or more of: outer abuse layers, sealant layers, tie layers, etc. In one embodiment, the front and back sheets include an outer abuse layer. During manufacturing, processing and shipping, the package, and hence the sheet material, may be exposed to environmental stresses, such as abrasion, high temperatures, and the like. As such, it may be desirable for the sheet material to include an outside or abuse layer that provides enhanced resistance to abuse. Further, since the abuse layer may be directly exposed to a heat seal bar of the heat-sealing equipment when forming the sterilizable package, the abuse layer preferably provides heat-resistant characteristics to the outer surfaces of the front and back sheets to help prevent "burn-through" during heat sealing. Suitable polymers for the abuse layer may include one or more of any of the following: polyolefins (e.g., polyethylenes, polypropylenes), polyamides, polyesters, polystyrenes, polyurethanes, and polycarbonates. Examples of suitable polyesters include amorphous (co)polyesters, poly(ethylene/terephthalic acid), and poly(ethylene/naphthalate). In a preferred embodiment, the front and back sheets include an outer abuse layer comprising polyester terephthalate.

The sheet material may also include a sealant layer on the opposite side of the sheet material from the abuse layer. The sealant layer typically defines an inner surface of the sterilizable package that faces the interior space of the package. The polymer material (i.e., component or blend of components) that forms the sealant layer has a melting point that facilitates heat sealing the inner surface of the sheet material to either itself, such as in the embodiment illustrated in FIGS. 1-3, or to a support member as shown in FIG. 4.

In one embodiment, the sheet material may comprise a multilayer laminate having an inner foil layer, such as aluminum foil. In this embodiment, the foil layer in addition to providing moisture and gas barrier properties also provides UV barrier properties. In a preferred embodiment, the sheet material comprises a laminate having an interior aluminum foil layer that is disposed between one or more polymeric layers. For example, a preferred laminate for use as the barrier film of the sheet material comprises a seven layer laminate having the following structure: an outer abuse layer comprising polyethylene terephthalate, a low density polyethylene layer, an inner aluminum foil layer, a nylon layer, a low density polyethylene layer and a sealant layer comprising low density polyethylene/ethylene vinyl acetate. In this embodiment, the low density polyethylene/ethylene vinyl acetate is heat sealable to itself or to the support member.

Sterilizable packages in accordance with the present invention may be prepared from a variety of suitable plastic materials whereby a strong, lightweight, reliable, yet economic package is provided. Preferably, the sheet material comprises a plastic material having an inner surface capable of forming a strong heat seal with itself or a support member. Additionally, packages for use in medical applications are generally formed from sheet material having both moisture barrier properties and gas barrier properties.

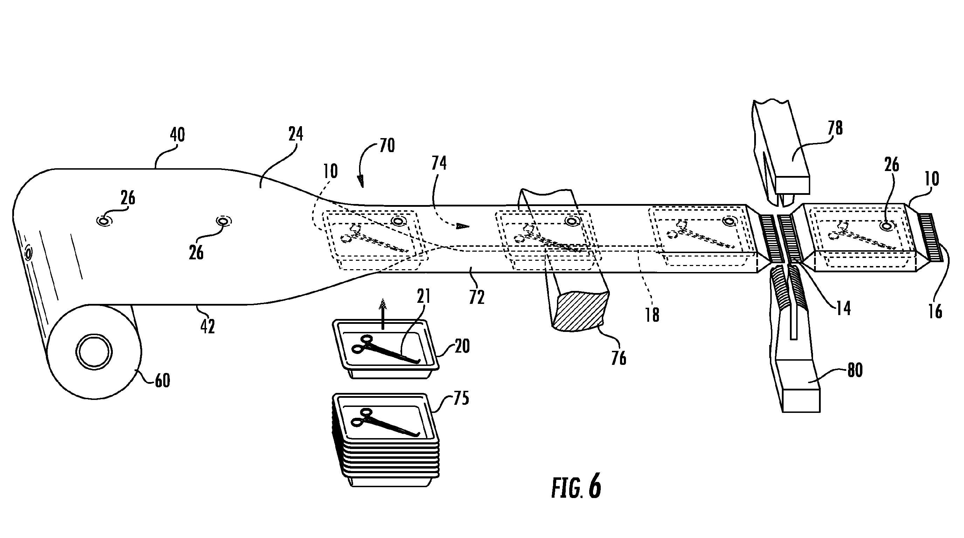

With reference to FIG. 6, an exemplary process and system for making the sterilizable package of FIG. 1 is illustrated in which a HFFS packaging system is illustrated. As shown, roll stock **60** of sheet material **24** having a plurality of breathable membranes **26** is provided. At **70** the sheet material is folded toward itself to form a generally tube like structure **72** having an interior space **74** for receiving support member **20** therein. Support member preloaded with article **21** is then inserted into the interior space of the thus formed tube. Preferably, support member **20** is provided from a supply **75** of a plurality of preloaded support members.

At heat sealing station **76,** a longitudinal heat seal extending in the machine direction of sheet material is formed joining the two opposing edges **40, 42** of the sheet material to each other and thereby form longitudinal seam **18.** Next, opposed heat sealing bars **78, 80** are used to heat seal the sheet material to itself to form seams **14, 16,** and thereby form the sterilizable package. In the illustrated embodiment, heat sealing bars **78, 80** are depicted as each including a pair heat sealing bar for simultaneously creating seams **14** and **16** on successive packages. However, it should be recognized that such seams cannot be created in separate steps.

After the packages are formed and sealed, a sterilizing gas is then introduced into the into the interior of the package through breathable membrane **26.** The sterilizing gas is introduced into the package for a sufficient amount of time so that the article is sterilized.

In some embodiments, the sterilizable package is flushed with an inert gas, such as nitrogen, prior to being filled. Additionally, a vacuum can also be applied to the interior of the sterilizable package prior to final sealing.

As should be evident from the foregoing discussion, the present invention can be used to package a wide variety of different items in which sterilization is desirable. In one particular embodiment, the present invention can be used to package a wide variety of medical devices and instruments including catheters, stents, and in particular drug coated stents.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A sterilizable package comprising:
- a pouch having two side seams on opposite ends of the pouch and a longitudinal seam extending therebetween, the pouch comprising a polymeric, barrier sheet material;
- a support member disposed in an interior space of the pouch, the support member having a support surface for supporting an article thereon;
- an opening formed in the sheet material and providing communication between the interior space of the pouch and an exterior environment of the pouch;
- a breathable membrane disposed on a surface of the sheet material and covering said opening, said breathable membrane having a peripheral edge overlying the sheet material and spaced from said side seams and longitudinal seam, the breathable membrane comprising a gas vapor permeable material; and
- a seam located at or adjacent the peripheral edge of the breathable membrane joining the membrane to the sheet material.

2. A sterilizable package comprising:
- a support member comprising a support surface and a periphery, the support surface for supporting an article thereon;
- a flexible, sheet material attached to the periphery of the support member with a continuous peripheral seam to thereby define a sealed sterilizable package having an interior space;
- an opening formed in the sheet material and providing communication between the interior space of the sterilizable package and outside environment of the sterilizable package;
- a breathable membrane disposed on a surface of the sheet material toward the interior space of the package covering said opening, said breathable membrane having a peripheral edge overlying the sheet material and spaced from said continuous peripheral seam, the breathable membrane comprising a gas permeable material; and
- a seam located at or adjacent the peripheral edge of the breathable membrane joining the membrane to the sheet material.

3. The sterilizable package of Claim 1 or 2, wherein the breathable membrane comprises paper or a nonwoven fabric.

4. The sterilizable package of Claim 1 or 2, wherein the inner surface of the sheet material comprises a heat sealable thermoplastic material.

5. The sterilizable package of Claim 1 or 2, wherein the breathable membrane covers about 10 to 50 percent of the surface of the sheet material.

6. The sterilizable package of Claim 1 or 2, wherein the breathable membrane has a surface area that is about 10 to 60 % larger than the surface area of said opening.

7. The sterilizable package of Claim 1 or 2, further comprising a medical instrument or medical device disposed in the interior space of the sterilizable package.

8. The sterilizable package of Claim 7, wherein the medical device is a stent that is coated with a therapeutic drug agent.

9. The sterilizable package of Claim 1, wherein the opening in the sheet material and the breathable membrane are circular, and are joined to each other by a continuous seam that is located at or adjacent the peripheral edge of the breathable membrane.

10. The sterilizable package of Claim 1, wherein the package is formed using a horizontal form fill seal process.

11. The sterilizable package of Claim 1, wherein the sheet material includes two longitudinal side edges, and wherein the sheet material is folded to form said opposing side seams of the pouch, and the two longitudinal side edges are sealed to each other to form said longitudinal seam that extends longitudinally along a length of the pouch.

12. The sterilizable package of Claim 2, wherein the support member comprises a thermoformed thermoplastic material.

13. A process of preparing a sterilizable package comprising:
- providing a polymeric sheet material having a pair of longitudinal side edges and a plurality of spaced apart openings through which a sterilizable gas can be transmitted from one side of the sheet material to the other, wherein a breathable membrane covers each of said openings, the breathable membrane comprising a gas permeable sheet material, and having a peripheral edge overlying the sheet material and being affixed to a surface of the sheet material with a seam located at or adjacent the peripheral edge of the breathable membrane, wherein the peripheral edge of the breathable membrane is spaced from said longitudinal side edges;
- folding the longitudinal side edges of the sheet material towards each other to form a tube-like structure having an interior space;
- inserting a support member into the interior space of said tube.
- sealing an inner surface of sheet material to itself to form a pair of opposing side seams and a longitudinal seam to thereby form a sealed sterilizable package.

14. The process of Claim 13, further comprising the step of placing a medical device or instrument on said support member.

15. The process of Claim 13, further comprising the step of introducing a sterilizing gas into the sterilizable package.
